# EUROPÄISCHE PATENTANMELDUNG

(11) **EP 1 201 262 A1**
(43) Veröffentlichungstag der Anmeldung: **02.05.2002**
(21) Anmeldenummer: 01125072.7
(22) Anmeldetag: 22.10.2001
(51) Int. Cl.: A61M 25/06

(54) **Hohlnadel**

(30) Priorität: 31.10.2000 DE 10053883
(71) Anmelder: Pulsion Medical Systems AG, 81829 München (DE)
(72) Erfinder: Pfeiffer, Ulrich J., Dr., 81667 München (DE); Thalmeier, Thomas, 85126 Münchsmünster (DE)
(74) Vertreter: Kehl, Günther, Dipl.-Phys.

(57) **Zusammenfassung**

Die Hohlnadel, welche an ihrer Spitze einen Anschliff (3) aufweist, erweitert sich in ihrem Außendurchmesser kontinuierlich von einem Bereich (1) nahe der Spitze jedoch hinter dem Anschliff (3) auf den etwa doppelten Außendurchmesser im Bereich (2) weiter entfernt von der Spitze. Der rotationssymmetrische Übergangsbereich (4) ist hierbei konisch und nach beiden Seiten zum spitznäheren bzw. spitzferneren Bereich (1,2) hin abgerundet.

## Beschreibung

Die vorliegende Erfindung betrifft eine Hohlnadel zur Punktion von Blutgefäßen und zur Einführung eines Drahtes, insbesondere für die Plazierung von Kathetern oder dergleichen.

Derartige Hohlnadeln, auch als Punktionskanülen bezeichnet, werden unter anderem eingesetzt bei der Applikation eines Katheters mittels sogenannter Seldinger-Technik. Hierbei wird in der Regel folgendermaßen vorgegangen: Das Blutgefäß, in welches der Katheter plaziert werden soll, wird mit der Hohlnadel punktiert. Durch die Hohlnadel wird ein Führungsdraht eingeführt, welcher im Gefäß verbleibt, während die Hohlnadel wieder herausgezogen wird. Bevor der Katheter auf den Führungsdraht gefädelt und über diesen in das Gefäß eingeführt wird, ist es für gewöhnlich erforderlich, die Punktionsstelle durch Einsatz eines sogennanten Dilatators zu erweitern. Ein Dilatator ist ein an einem Ende konisch zulaufender Kunststoffschlauch, der über den Führungsdraht an die Punktionsstelle geführt wird, um sie auf den Außendurchmesser des Katheters aufzuweiten.

Vor dem Hintergrund der generellen Anforderung im medizinischen Bereich, jedweden Eingriff am menschlichen Körper möglichst schonend und risikoarm durchzuführen, nicht unnötig in die Länge zu ziehen, und entsprechend die Zahl vermeidbarer Schritte eines Eingriffs zu verringern, liegt der Erfindung die Aufgabe zugrunde, eine Vorrichtung zu schaffen, deren Anwendung das Plazieren eines Katheters in Blutgefäßen verschnellert und erleichtert, ohne auf die Vorteile der Anwendung eines herkömmlichen Dilatators verzichten zu müssen.

Diese Aufgabe wird gemäß der vorliegenden Erfindung durch eine Hohlnadel zur Punktion von Blutgefäßen und zur Einführung eines Drahtes, insbesondere für die Plazierung von Kathetern oder dergleichen, gelöst, welche in einem ersten Bereich nahe ihrer Spitze einen ersten Außendurchmesser und in einem zweiten Bereich, welcher weiter von der Spitze entfernt ist als der erste Bereich, einen zweiten Außendurchmesser aufweist, wobei der zweite Außendurchmesser größer ist als der erste Außendurchmesser, vorzugsweise um das 1,2 bis 3,0-fache. Eine solche Nadel bietet den Vorteil, daß das Punktieren und schonende Aufweiten der Punktionsstelle in einem Arbeitsschritt vonstatten geht. Dabei bleiben die Vorteile eines herkömmlichen Dilatators erhalten, ohne daß ein solcher eingesetzt wird. Durch das Entfallen des Dilatators ist ein Hilfsmittel weniger anzuschaffen, aufzubewahren und zu sterilisieren, so daß sich neben dem Aufwand für den eigentlichen Eingriff auch der begleitende Aufwand reduziert.

Vorzugsweise weist die Nadel im Bereich ihrer Spitze einen Anschliff auf.

In einer vorteilhaften Ausführungsform der vorliegenden Erfindung liegt der erste Außendurchmesser zwischen 0,4 und 1,5 mm.

Vorzugsweise erweitert sich der Querschnitt der Hohlnadel in einem Übergangsbereich vom ersten Außendurchmesser zum zweiten Außendurchmesser kontinuierlich. Günstig ist eine konische Form des Übergangsbereichs wobei im konischen Bereich der Öffnungswinkel zwischen der longitudinalen Achse der Hohlnadel und einer, in einer gemeinsamen Ebene liegenden Mantellinie vorzugsweise zwischen 10° und 45° liegt.

Vorteilhafterweise beginnt der Übergangsbereich hinter dem Anschliff.

Günstig wirkt sich ein abgerundeter Übergang zwischen dem ersten und dem zweiten Außendurchmesser aus.

In einer besonders bevorzugten Ausführungsform der vorliegenden Erfindung sind die Bereiche mit dem ersten und zweiten Außendurchmesser und der Übergangsbereich einstückig ausgebildet. Hierdurch kommt der verminderte Aufwand gegenüber der Verwendung eines Dilatators als zusätzliches Hilfsmittel besonders zum tragen.

Nachstehend wird ein Ausführungsbeispiel der vorliegenden Erfindung anhand der schematischen, nicht maßstäblichen Zeichnungen erläutert. Hierbei zeigt:
- Fig.1:: eine schematische Ansicht des vorderen Teils einer erfindungsgemäßen Hohlnadel,
- Fig.2:: einen Längsschnitt durch den in Fig.1 dargestellten vorderen Teil einer erfindungsgemäßen Hohlnadel, wobei die Nadel um 90 Grad um ihre Längsachse gedreht ist.

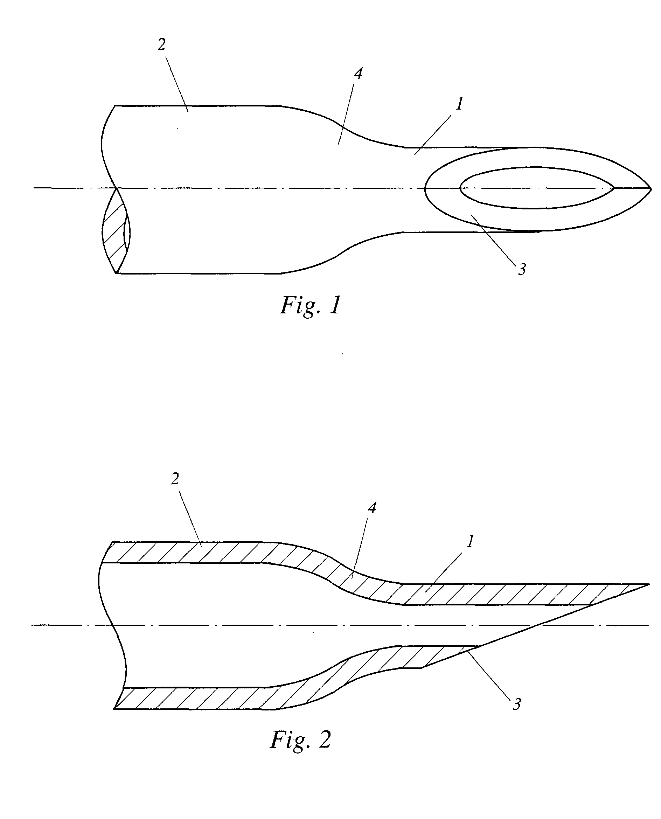

Die in Fig.1 und Fig.2 dargestellte Hohlnadel, welche an ihrer Spitze einen Anschliff 3 aufweist, erweitert sich in ihrem Außendurchmesser kontinuierlich von einem Bereich 1 nahe der Spitze jedoch hinter dem Anschliff 3 auf den etwa doppelten Außendurchmesser im Bereich 2 weiter entfernt von der Spitze. Der rotationssymmetrische Übergangsbereich 4 ist hierbei konisch und nach beiden Seiten zum spitznäheren bzw. spitzferneren Bereich 1, 2 hin abgerundet.

In der Anwendung schneidet sich der Spitzanschliff 3 der Kanüle durch die Haut, das zwischenliegende Gewebe und die Gefäßwand. Der aufgeweitete Teil 2 der Hohlnadel erweitert die Punktionsstelle. Somit werden in einem einzigen Arbeitsschritt die Vorteile erzielt, die bei Verwendung herkömmlicher Punktionskanülen erst durch den zusätzlichen Einsatz eines Dilatators gewonnen werden: Durch eine Primärpunktion mit geringem Durchmesser und Aufweitung des Punktionsloches auf den erforderlichen Durchmesser zur Einführung eines entsprechend dicken Katheters bildet sich letztlich die traumatische Schädigung des Blutgefäßes relativ rasch wieder auf den kleineren Punktionsdurchmesser zurück. Damit bleibt gegenüber der Verwendung einer insgesamt dickeren Nadel mit nur einem Außendurchmesser die Zeit der Wundheilung auf die eines kleineren Loches beschränkt. Der Heilungsprozeß verkürzt sich demnach und der Patient wird weitestmöglich geschont.

## Patentansprüche

1. Hohlnadel zur Punktion eines Blutgefäßes und Aufweitung der Punktionsstelle im selben Arbeitsgang zur Einführung eines Drahtes für die Plazierung eines Katheters, wobei die Nadel in einem ersten Bereich nahe ihrer Spitze (1) einen ersten Außendurchmesser aufweist und in einem zweiten Bereich (2), welcher weiter von der Spitze entfernt ist als der erste Bereich (1), einen zweiten Außendurchmesser aufweist, der größer ist als der erste Außendurchmesser.

2. Hohlnadel nach Anspruch 1, welche im Bereich der Spitze einen Anschliff (3) aufweist.

3. Hohlnadel nach einem der vorherigen Ansprüche, wobei das Verhältnis vom zweiten Außendurchmesser zum ersten Außendurchmesser zwischen 1,2 und 3,0 liegt.

4. Hohlnadel nach einem der vorherigen Ansprüche, wobei der erste Außendurchmesser zwischen 0,4 mm und 1,5 mm liegt.

5. Hohlnadel nach einem der vorherigen Ansprüche, wobei sich der Querschnitt der Nadel vom ersten Außendurchmesser zum zweiten Außendurchmesser in einem Übergangsbereich (4) kontinuierlich erweitert.

6. Hohlnadel nach Anspruch 5, welche im Übergangsbereich (4) eine konische Form aufweist.

7. Hohlnadel nach Anspruch 6, wobei der Öffnungswinkel des konischen Übergangsbereichs (4) zwischen 10° und 45° liegt.

8. Hohlnadel nach einem der Ansprüche 5 bis 7, wobei der Übergangsbereich (4) hinter dem Anschliff (3) beginnt.

9. Hohlnadel nach einem der Ansprüche 5 bis 8, wobei der Übergang (4) zwischen den ersten und zweiten Außendurchmessern abgerundet ist.

10. Hohlnadel nach einem der vorherigen Ansprüche, wobei der erste Bereich (1) und der zweite Bereiche (2) mit den ersten und zweiten Außendurchmessern und der Übergangsbereich (4) einstückig ausgebildet sind.

11. Verwendung einer Hohlnadel zur Punktion eines Blutgefäßes und Aufweitung der Punktionsstelle im selben Arbeitsgang zur Einführung eines Drahtes für die Plazierung eines Katheters, wobei die Nadel in einem ersten Bereich nahe ihrer Spitze (1) einen ersten Außendurchmesser aufweist und in einem zweiten Bereich (2), welcher weiter von der Spitze entfernt ist als der erste Bereich (1), einen zweiten Außendurchmesser aufweist, der größer ist als der erste Außendurchmesser.

12. Verwendung einer Hohlnadel nach Anspruch 11, wobei die Hohlnadel im Bereich der Spitze einen Anschliff (3) aufweist.

13. Verwendung einer Hohlnadel nach einem der Ansprüche 11-12, wobei das Verhältnis vom zweiten Außendurchmesser zum ersten Außendurchmesser zwischen 1,2 und 3,0 liegt.

14. Verwendung einer Hohlnadel nach einem der Ansprüche 11-13, wobei der erste Außendurchmesser zwischen 0,4 mm und 1,5 mm liegt.

15. Verwendung einer Hohlnadel nach einem der Ansprüche 11-14, wobei sich der Querschnitt der Nadel vom ersten Außendurchmesser zum zweiten Außendurchmesser in einem Übergangsbereich (4) kontinuierlich erweitert.

16. Verwendung einer Hohlnadel nach Anspruch 15, die im Übergangsbereich (4) eine konische Form aufweist.

17. Verwendung einer Hohlnadel nach Anspruch 16, wobei der Öffnungswinkel des konischen Übergangsbereichs (4) zwischen 10° und 45° liegt.

18. Verwendung einer Hohlnadel nach einem der Ansprüche 15-17, wobei der Übergangsbereich (4) hinter dem Anschliff (3) beginnt.

19. Verwendung einer Hohlnadel nach einem der Ansprüche 15-18, wobei der Übergang (4) zwischen den ersten und zweiten Außendurchmessern abgerundet ist.

20. Verwendung einer Hohlnadel nach einem der Ansprüche 11-19, wobei der erste Bereich (1) und der zweite Bereiche (2) mit den ersten und zweiten Außendurchmessern und der Übergangsbereich (4) einstückig ausgebildet sind.
